Europäisches Patentamt

European Patent Office

Office européen des brevets

(11) Veröffentlichungsnummer: **0 270 744 B1**

# (12) EUROPÄISCHE PATENTSCHRIFT

(45) Veröffentlichungstag der Patentschrift: **02.05.91**

(21) Anmeldenummer: **87112260.2**

(22) Anmeldetag: **24.08.87**

(51) Int. Cl.5: **A61F 2/34**

(54) **Hüftpfannen-Endoprothese.**

(30) Priorität: **10.11.86 DE 8630015 U**

(43) Veröffentlichungstag der Anmeldung:
**15.06.88 Patentblatt 88/24**

(45) Bekanntmachung des Hinweises auf die
Patenterteilung:
**02.05.91 Patentblatt 91/18**

(84) Benannte Vertragsstaaten:
**CH DE ES FR GB IT LI SE**

(56) Entgegenhaltungen:
**WO-A-85/00284**
**FR-A- 2 197 561**
**US-A- 4 241 463**

(73) Patentinhaber: **Waldemar Link GmbH & Co**
**Barkhausenweg 10**
**W-2000 Hamburg 63(DE)**

(72) Erfinder: **Link, Helmut D.**
**Wildstieg 14**
**W-2000 Hamburg 65(DE)**
Erfinder: **Keller, Arnold**
**An der Naherfurth 5**
**W-2061 Kayhude(DE)**

(74) Vertreter: **Glawe, Delfs, Moll & Partner Patentanwälte**
**Postfach 26 01 62 Liebherrstrasse 20**
**W-8000 München 26(DE)**

## Beschreibung

Die Erfindung bezieht sich auf eine Hüftpfannen-Endoprothese bestehend aus einer mittels eines an ihrer Außenseite vorgesehenen Gewindes implantierbaren Fassung und einem die Gelenkfläche bildenden, in der Fassung verankerbaren Einsatz.

Bei bekannten Schraubpfannen dieser Art (DE-OS 24 11 617; DE-OS 33 25 448) sowie bei mit Knochenzement zu verankernden Hüftpfannen (US-PS-4,380,090) ist die Fassung (abgesehen vom Gewinde und den Halterungsmitteln für den Einsatz) als Rotationskörper mit kreisförmigem Rand ausgebildet. Demgegenüber ist der Rand der knöchernen Gelenkpfanne nicht kreisförmig; vielmehr tritt er in seinem unteren, schräg nach innen gerichteten Teil ein wenig zurück. Weil man dies nur beschränkt bei der Einstellung des Prothesensitzes berücksichtigen kann, kann es in diesem Bereich bei Verwendung einer Prothese mit kreisförmig geschlossenem Rand zu Beschwerden kommen, weil nicht genügend Platz für Weichteile und Nervensträge zur Verfügung steht. Zwar ist es bekannt (DE-PS 18 06 323), eine mit Zement zu verankernde Hüftpfanne in diesem Bereich auszuschneiden; bei einer Schraubpfanne ist dies aber nicht möglich, weil man vor ihrem Einsetzen nicht mit Sicherheit weiß, in welcher Rotationsstellung die endgültige Verankerung erreicht wird.

Ferner ist eine Hüftgelenkendoprothese bekannt (DE-A-32 00 340), welche eine Gelenkpfanne und einen an der Gelenkpfanne zu befestigenden Haltering umfaßt, wobei der Haltering eine unterschiedliche Höhe zum Zwecke einer ausreichenden Bewegungsmöglichkeit des Gelenks aufweist. Auch in diesem Falle wird die Gelenkpfanne mittels Knochenzement im Acetabulum verankert; ihre Stellung bezüglich des Beckenknochens kann somit vor der Verankerung bestimmt werden.

Der Erfindung liegt daher die Aufgabe zugrunde, eine Hüftpfannen-Endoprothese der eingangs genannten Art zu schaffen, die als Schraubpfanne mit einem an bestimmter Stelle zu positionierenden Ausschnitt versehen werden kann.

Die erfindungsgemäße Lösung besteht darin, daß die Endoprothese einen in unterschiedlichen Drehstellungen an die öffnungsseitige Stirnfläche der Fassung ansetzbaren, zu einem Teilring einseitig ausgeschnittenen Ringteil aufweist, oder darin, daß die Endoprothese die Kennzeichnenden Merkmale des Anspruchs 3 aufweist.

Unabhängig von der Drehstellung, in welcher die Fassung ihre endgültige Verankerung erreicht, kann der ausgeschnittene Ringteil in solcher Winkellage angesetzt werden, daß der Ausschnitt sich an der gewünschten Stelle befindet. Dies gilt auch dann, wenn die Fassung und der Ringteil mit zusammenwirkenden Drehverhinderungsmitteln versehen sind. Diese können nämlich so ausgebildet sein, daß der ausgeschnittene Ringteil in unterschiedlichen Drehstellungen an die Fassung ansetzbar ist. Alternativ können mehrere wahlweise verwendbare, zu einem Teilring einseitig ausgeschnittene Ringteile mit unterschiedlicher Lage des Ausschnitts vorgesehen sein, wobei der Ringteil an die öffnungsseitige Stirnfläche der Fassung ansetzbar ist.

Zweckmäßigerweise ist der ausgeschnittene Ringteil unmittelbar an der Fassung befestigbar. Nach einer anderen Lösung kann er aber auch mittelbar von dem an der Fassung befestigbaren Einsatz gehalten sein. Er ist zweckmäßigerweise ein vom Einsatz gesonderter Teil, kann aber auch einstückig mit diesem ausgebildet sein. Im letzteren Fall weist der Einsatz einen Randteil auf, der gegenüber der vom Einsatz aufgenommenen und mit diesem passend zusammenwirkenden Außenfläche stufig nach außen vorspringt. Außerhalb des Ausschnitts kann seine Umfangsfläche die Außenfläche der Fassung mehr oder weniger fortsetzen. Er liegt mit dieser Außenfläche der Innenfläche des Hüftpfanneneingangs unmittelbar an. Dies hindert nicht, daß außerdem noch in bekannter Weise ein seitlich vorkragender Flanschteil vorhanden ist, der mit einer lotrecht zur Fassungsachse verlaufenden Fläche außerhalb des Hüftpfanneneingangs am Knochen anliegt.

Nach einem weiteren Merkmal der Erfindung kann zusätzlich ein nicht ausgeschnittener, die Fassung zur Gestalt herkömmlicher Prothesen ergänzender Ringteil vorgesehen sein, damit die Prothese mittels des herkömmlichen Instrumentariums eingesetzt werden kann. Dieser nicht ausgeschnittene Ringteil besitzt zweckmäßigerweise dieselbe Außengestalt wie der ausgeschnittene Ringteil außerhalb seines Ausschnitts. Er ermöglicht es, schon während der Montage der Fassung die Anlageverhältnisse für den ausgeschnittenen Ringteil passend vorzubereiteten bzw. zu überprüfen. Dies gilt auch dann, wenn der ausgeschnittene Ringteil einstückig mit dem Einsatz ausgebildet ist.

Der ausgeschnittene Ringteil ergibt einerseits außerhalb seines Ausschnitts die gewünschte Abstützung für den Einsatz am Knochen bzw. an der Fassung, während er im Bereich des Ausschnitts die gewünschte Bewegungsfreiheit zur Verfügung stellt.

Es kommt im Zusammenhang der Erfindung nicht darauf an, ob der Ringteil im Bereich des Ausschnitts vollständig unterbrochen ist oder ob sein Querschnitt lediglich zur Öffnungsseite der Prothese hin vermindert ist. Zweckmäßigerweise ist auch der Einsatz entsprechend ausgeschnitten. Unter einem Ausschnitt ist alles zu verstehen, was den öffnungsseitigen Rand der Fassung bzw. des Ringteils bzw. des Einsatzes gegenüber der sonstigen Öffnungsrandebene des Einsatzes oder der Fassung zurückverlegt, insbesondere auch hinter die Ebene, in der die nicht ausgeschnitten Teile des Einsatzes oder des ausgeschnittenen Ringteils mit einem seitlich vorragenden Öffnungsflansch am Knochen anliegen.

Die Erfindung wird im folgenden näher unter Bezugnahme auf die Zeichnung erläutert, die ein vorteilhaftes Ausführungsbeispiel veranschaulicht. Darin zeigen:

Fig. 1 einen Querschnitt durch die gesamte Prothese,

Fig. 2 und 3 eine Draufsicht und teilweise geschnittene Seitenansicht der Fassung und

Fig. 4 und 5 eine Schnittansicht und eine Draufsicht des Ringteils.

Die töpfchenförmige Fassung (1) bildet auf der Innenseite eine Konusfläche (2) zum Halten des Einsatzes (3) und weist auf ihrer Außenseite Gewindegänge (4) zur Schraubbefestigung in einer Ausfräsung in der knöchernen Hüftpfanne auf. Zur Verhinderung gegenseitiger Drehung ist am Boden der Fassung ein Stift (5) vorgesehen, während der Einsatz (3) auf einem entsprechenden Kreis eine Vielzahl dazu passender Bohrungen aufweist, die bei unterschiedlicher Rotationsstellung des Einsatzes zur Fassung wahlweise mit dem Stift (5) in Eingriff gebracht werden können. Der Einsatz (3) besitzt am Boden Rastnasen (6), die hinter einen entsprechenden Bodenflansch der Fassung fassen, um den Einsatz in der vorgesehenen Stellung zu sichern. Die Gelenkfläche des Einsatzes ist bei (7) angegeben. Insoweit kann die Anordnung als bekannt angesehen werden.

Die Fassung (1) ist vom Boden bis zu ihrer öffnungsseitigen Stirnfläche (8) weniger tief als bekannte ausgeführt. Die Höhendifferenz wird von dem Ringteil (9) ausgeglichen, der passend in geeigneter Weise an den Öffnungsrand der Fassung (1) angesetzt werden kann und die Innenfläche (2) der Fassung passend zum Einsatz (3) fortsetzt. Er weist am Umfang Federvorsprünge (10) auf, die zueinander um 90° versetzt sind und in Nuten (11) passen, die um 45° zueinander versetzt am Innenrand der Fassung (1) angeordnet sind. Der Ring (9) kann daher in unterschiedlichen, jeweils um 45° zueinander versetzten Rotationsstellungen mit der Fassung (1) verbunden werden. Selbstverständlich kann die Winkelanordnung der Federvorsprünge und Nuten auch anders gewählt sein, um beispielsweise eine feinstufigere Einstellbarkeit zu ermöglichen.

Die öffnungsseitige Stirnfläche (12) des Ringteils (9) entspricht derjenigen herkömmlicher Fassungen und stützt den Rand (13) des Einsatzes (3) ab. Die Außenform des Ringteils (9) entspricht im übrigen dem Rand herkömmlicher Fassungen und stützt sich im implantierten Zustand vorzugsweise am Hüftpfanneneingang des Knochens ab. Sie bildet eine im wesentlichen parallel zur Prothesenachse verlaufende Umfangsfläche, die an einer entsprechenden, nach innen gewendeten Fläche des Knochens anliegen kann, sowie einen seitlich ein wenig vorragenden Flansch, dessen Rückfläche sich an einer in der Öffnungsebene der Hüftpfanne liegenden Fläche des Knochens anlegen kann.

Der Ringteil (9) ist im Bereich (14) ausgeschnitten um vorzugsweise etwa 90 bis 150°, im dargestellten Beispiel etwa 120°. Trotzdem wird der Einsatz (3) hinreichend von der Fassung und dem Ringteil (9) gehalten, weil sich der Ausschnitt in einem weniger belasteten Bereich der Prothese befindet.

Zur Implantation der Fassung kann der Ringteil (9) durch einen nicht ausgeschnittenen Ringteil ersetzt sein, der die Gestalt der Fassung zu derjenigen herkömmlicher Fassungen ergänzt und dadurch die Benutzung herkömmlichen Instrumentariums ermöglicht. Statt dessen kann auch spezielles Instrumentarium verwendet werden, das die Benutzung des unausgeschnittenen Rings nicht verlangt und ggf. selbst mit einem entsprechenden Ringteil versehen ist, der die Anpassung des Knochens an den ausgeschnittenen Ring (9) bzw. die Überprüfung der Anlageverhältnisse ermöglicht.

Im dargestellten Beispiel sind keine unmittelbar zusammenwirkenden Befestigungsmittel am ausgeschnittenen Ringteil (9) und an der Fassung (1) vorgesehen. Vielmehr wird der Ringteil (9) von dem Einsatz (13) gehalten, der seinerseits mittels der Rasteinrichtungen (6) an der Fassung (1) befestigt ist.

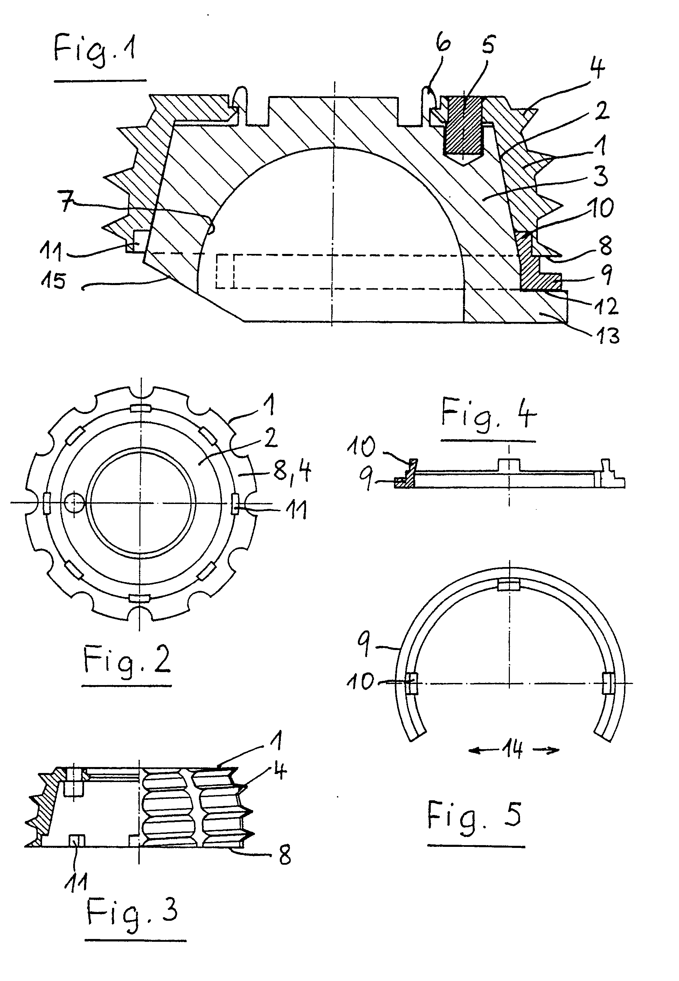

Wenn der ausgeschnittene Ringteil einstückig von dem Einsatz gebildet ist, hat man sich in Fig. 1 die Querschnittsteile (9) und (10) in den Querschnitt des Einsatzes einbezogen vorzustellen. Der den ausgeschnittenen Ring bildende Teil des Einsatzes ist dann einerseits an dem Ausschnitt und andererseits an seiner über die Konusfläche (2) stufig hinausgehende Außenfläche erkennbar, die unmittelbar am Knochen anliegt.

Der Einsatz (3) ist im Bereich des Ausschnitts (14) ebenfalls, wie bei (15) gezeigt, ausgeschnitten.

**Ansprüche**

1. Hüftpfannen-Endoprothese bestehend aus einer mittels eines an ihrer Außenseite vorgesehenen Gewindes (4) implantierbaren Fassung (1) und einem die Gelenkfläche (7) bildenden, in der Fassung (1) verankerbaren Einsatz (3), dadurch gekennzeichnet, daß die Endoprothese einen in unterschiedlichen Drehstellungen an die öffnungsseitige Stirnfläche (8) der Fassung (1) ansetzbaren, zu einem Teilring einseitig ausgeschnittenen Ringteil (9) aufweist.

2. Hüftpfannen-Endoprothese nach Anspruch 1, dadurch gekennzeichnet, daß die Fassung (1) und der Ringteil (9) mit zusammenwirkenden Drehverhinderungsmitteln (10, 11) versehen sind.

3. Hüftpfannen-Endoprothese bestehend aus einer mittels eines an ihrer Außenseite vorgesehenen Gewindes (4) implantierbaren Fassung (1) und einem die Gelenkfläche (7) bildenden, in der Fassung (1) verankerbaren Einsatz (3), dadurch gekennzeichnet, daß die Endoprothese mehrere wahlweise verwendbare, zu einem Teilring einseitig ausgeschnittene Ringteile (9) mit unterschiedlicher Lage des Ausschnitts (14) aufweist, wobei der Ringteil (9) an die öffnungsseitige Stirnfläche (8) der Fassung (1) ansetzbar ist.

4. Hüftpfannen-Endoprothese nach einem der Ansprüche 1 bis 3, dadurch gekennzeichnet, daß der ausgeschnittene Ringteil (9) unmittelbar an der Fassung (1) befestigbar ist.

5. Hüftpfannen-Endoprothese nach einem der Ansprüche 1 bis 4, dadurch gekennzeichnet, daß der ausgeschnittene Ringteil (9) mittelbar von dem an der Fassung (1) befestigbaren Einsatz (3) gehalten ist.

6. Hüftpfannen-Endoprothese nach einem der Ansprüche 1 bis 5, dadurch gekennzeichnet, daß der ausgeschnittene Ring (9) ein vom Einsatz (3) gesonderter Teil ist.

7. Hüftpfannen-Endoprothese nach einem der Ansprüche 1 bis 5, dadurch gekennzeichnet, daß der ausgeschnittene Ring einstückig mit dem Einsatz ausgebildet ist.

8. Hüftpfannen-Endoprothese nach einem der Ansprüche 1 bis 7, dadurch gekennzeichnet, daß außerdem ein nicht ausgeschnittener, die Fassung (1) zur Gestalt herkömmlicher Prothesen ergänzender Ringteil vorgesehen ist.

9. Hüftpfannen-Endoprothese nach Anspruch 8, dadurch gekennzeichnet, daß die Außenform des nicht ausgeschnittenen Ringteils mit dem ausgeschnittenen Ringteil außerhalb von dessen Ausschnitt (14) übereinstimmt.

**Claims**

1. An endoprosthesis for the cotyloid cavity, comprising a socket member (1), which can be implanted by means of a screw thread (4) provided on its exterior, and an insert (3) which forms the articulation surface (7) and which can be anchored in the socket member (1), characterised in that the endoprosthesis has an annular portion (9) which in different rotational positions can be applied against the end surface (8) on the opening side of the socket member (1) and which on one side is cut away to form a part of a ring.

2. An endoprosthesis for the cotyloid cavity according to Claim 1, characterised in that the socket member (1) and the annular portion (9) are provided with co-operating means (10, 11) for preventing rotation.

3. An endoprosthesis for the cotyloid cavity, comprising a socket member (1), which can be implanted by means of a screw thread (4) provided on its exterior, and an insert (3) which forms the articulation surface (7) and which can be anchored in the socket member (1), characterised in that the endoprosthesis has a plurality of alternatively usable annular portions (9) cut away on one side to form a part of a ring and with differing position of the cut-away portion (14), wherein the annular portion (9) can be applied against the end surface (8) on the opening side of the socket member (1).

4. An endoprosthesis for the cotyloid cavity according to any one of Claims 1 to 3, characterised in that the cutaway annular portion (9) can be secured directly to the socket member (1).

5. An endoprosthesis for the cotyloid cavity according to any one of Claims 1 to 4, characterised in that the cut-away annular portion (9) is retained indirectly by the insert (3) which can be secured to the socket member (1).

6. An endoprosthesis for the cotyloid cavity according to any one of Claims 1 to 5, characterised in that the cut-away ring (9) is a separate part from the insert (3).

7. An endoprosthesis for the cotyloid cavity according to any one of Claims 1 to 5, characterised in that the cutaway ring is formed in one piece with the insert.

8. An endoprosthesis for the cotyloid cavity according to any one of Claims 1 to 7, characterised in that additionally an annular portion is provided which is not cut away and which completes the socket member (1) to form a Conventional prosthesis.

9. An endoprosthesis for the cotyloid cavity according to Claim 8, characterised in that the exterior shape of the annular portion which is not cut-away corresponds to the annular portion which is cut-away outside the cut-away portion (14) thereof.

## Revendications

1. Endoprothèse cotyloïdienne, se composant d'une douille (1) implantable au moyen d'un filetage (4) prevu sur sa surface extérieure et d'un insert (3) qui est ancrable dans la douille (1) et que forme la surface articulaire (7), caractérisée en ce que l'endoprothèse comporte une pièce annulaire (9) qui est ouverte d'un côté en un anneau partiel et qui peut être appliquée dans différentes positions angulaires sur la surface d'extrémité (8) du côté ouvert de la douille (1).

2. Endoprothèse cotyloïdienne selon la revendication 1, caractérisee en ce que la douille (1) et la Pièce annulaire (9) sont munies de moyens (10), 11) qui coopèrent pour les empêcher de tourner l'une par rapport à l'autre.

3. Endoprothèse cotyloïdienne, se composant d'une douille (1) implantable au moyen d'un filetage (4) prevu sur sa surface extérieure et d'un insert (3) qui est ancrable dans la douille (1) et qui forme la surface articulaire (7), caractérisée en ce que l'endoprothèse comporte plusieurs pièces annulaires (9) utilisables au choix, qui sont ouvertes d'un côté en un anneau partiel avec une position différente de leur ouverture (14), la pièce annulaire (9) pouvant être appliquée sur la surface d'extrémité (8) du côté ouvert de la douille (1).

4. Endoprothèse cotyloïdienne selon l'une quelconque des revendications 1 à 3, caractérisée en ce que la pièce annulaire ouverte (9) peut être fixée directement sur la douille (1).

5. Endoprothèse cotyloïdienne selon l'une quelconque des revendications 1 à 4, caractérisée en ce que la pièce annulaire ouverte (9) est maintenue indirectement par l'insert (3) qui peut être fixé à la douille (1).

6. Endoprothèse cotyloïdienne selon l'une quelconque des revendications 1 à 5, caractérsée en ce que l'anneau ouvert (9) est une pièce distincte de l'insert (3).

7. Endoprothèse cotyloïdienne selon l'une quelconque des revendications 1 à 5, caractérisée en ce que l'anneau ouvert est forme d'une seule pièce avec l'insert.

8. Endoprothèse cotyloïdienne selon l'une quelconque des revendications 1 à 7, caractérisé en ce qu'il est prevu en outre une pièce annulaire non ouverte qui complète la douille (1) pour lui donner la forme de prothèses traditionnelles.

9. Endoprothèse cotyloïdienne selon la revendication 8, caractérisée en ce que la forme extérieure de la pièce annulaire non ouverte correspond à celle de la pièce annulaire ouverte en dehors de l'ouverture (14) de celle-ci.

Fig.1

6
5
4
2
1
3
10
8
9
12
13
7
11
15

Fig. 2

1
2
8,4
11

Fig.3

1
4
8
11

Fig. 4

10
9

Fig. 5

9
10
14